# EUROPÄISCHE PATENTSCHRIFT

(11) **EP 2 171 081 B1**
(45) Veröffentlichungstag und Bekanntmachung des Hinweises auf die Patenterteilung: **29.02.2012**
(21) Anmeldenummer: 08774450.4
(22) Anmeldetag: 27.06.2008
(51) Int. Cl.: C12Q 1/02, G01N 33/543, G01N 33/58

(54) **EINRICHTUNG UND VERFAHREN ZUM NACHWEIS EINER SUBSTANZ MITTELS PARTIKEL-PLASMONEN- RESONANZ (PPR) ODER PARTIKEL-VERMITTELTER FLUORESZENZ AUF DER BASIS VON ZELLOBERFLÄCHENPOLARISIERUNGEN**
DEVICE AND METHOD FOR DETECTING A SUBSTANCE BY MEANS OF PARTICLE PLASMON RESONANCE (PPR) OR PARTICLE-MEDIATED FLUORESCENCE BASED ON CELL SURFACE POLARIZATIONS
DISPOSITIF ET PROCEDE POUR METTRE EN EVIDENCE UNE SUBSTANCE AU MOYEN DE LA RESONANCE PLASMONIQUE DES PARTICULES (PPR) OU DE LA FLUORESCENCE INDUITE PAR LES PARTICULES SUR LA BASE DE POLARISATIONS DE SURFACE CELLULAIRE

(30) Priorität: 27.06.2007 DE 102007031533
(43) Veröffentlichungstag der Anmeldung: 07.04.2010
(73) Patentinhaber: Technische Universität Dresden, 01069 Dresden (DE)
(72) Erfinder: OSTERMANN, Kai, 01187 Dresden (DE); POMPE, Wolfgang, 01737 Hartha (DE); WERSING, Dagmar, 01097 Dresden (DE); LAKATOS, Mathias, 01067 Dresden (DE); MERTIG, Michael, 01169 Dresden (DE); RÖDEL, Gerhard, 85757 Karlsfeld (DE); THIERFELDER, Simone, 01237 Dresden (DE)
(74) Vertreter: Kailuweit & Uhlemann Patentanwälte
(86) Internationale Anmeldenummer: PCT/EP2008/058288
(87) Internationale Veröffentlichungsnummer: WO 2009/000920

(56) Entgegenhaltungen:
- US-A1- 2003 211 615
- RON ET AL: "Biosensing environmental pollution" CURRENT OPINION IN BIOTECHNOLOGY, LONDON, GB, Bd. 18, Nr. 3, 8. Juni 2007 (2007-06-08), Seiten 252-256, XP022110189 ISSN: 0958-1669
- BENTON ET AL: "The utilization of a Saccharomyces cerevisiae HUG1P-GFP promoter-reporter construct for the selective detection of DNA damage" MUTATION RESEARCH. GENETIC TOXICOLOGY AND ENVIRONMENTALMUTAGENESIS, ELSEVIER, AMSTERDAM, NL, Bd. 633, Nr. 1, 13. Mai 2007 (2007-05-13), Seiten 21-34, XP022170046 ISSN: 1383-5718
- BELKIN S: "MICROBIAL WHOLE-CELL SENSING SYSTEMS OF ENVIRONMENTAL POLLUTANTS" CURRENT OPINION IN MICROBIOLOGY, CURRENT BIOLOGY LTD, GB, Bd. 6, Nr. 3, 1. Juni 2003 (2003-06-01), Seiten 206-212, XP001202139 ISSN: 1369-5274
- PROSZYNSKI TOMASZ J ET AL: "Plasma membrane polarization during mating in yeast cells." THE JOURNAL OF CELL BIOLOGY 19 JUN 2006, Bd. 173, Nr. 6, 19. Juni 2006 (2006-06-19), Seiten 861-866, XP002497144 ISSN: 0021-9525
- AARON JESSE ET AL: "Plasmon resonance coupling of metal nanoparticles for molecular imaging of carcinogenesis in vivo" JOURNAL OF BIOMEDICAL OPTICS, Bd. 12, Nr. 3, Mai 2007 (2007-05), Seite Article No.: 034007, XP002497145 ISSN: 1083-3668
- GEORGE NATHALIE ET AL: "SPECIFIC LABELING OF CELL SURFACE PROTEINS WITH CHEMICALLY DIVERSE COMPOUNDS" JOURNAL OF THE AMERICAN CHEMICAL SOCIETY, AMERICAN CHEMICAL SOCIETY, WASHINGTON, DC.; US, US, Bd. 126, Nr. 29, 28. Juli 2004 (2004-07-28), Seiten 8896-8897, XP009079519 ISSN: 0002-7863
- GITAI ZEMER ET AL: "An actin-like gene can determine cell polarity in bacteria.", PROCEEDINGS OF THE NATIONAL ACADEMY OF SCIENCES OF THE UNITED STATES OF AMERICA 8 JUN 2004 LNKD- PUBMED:15159537, vol. 101, no. 23, 8 June 2004 (2004-06-08) , pages 8643-8648, ISSN: 0027-8424
- LÖRINCZ A T ET AL: "Sequence analysis of temperature-sensitive mutations in the Saccharomyces cerevisiae gene CDC28.", MOLECULAR AND CELLULAR BIOLOGY NOV 1986 LNKD- PUBMED:3540606, vol. 6, no. 11, November 1986 (1986-11), pages 4099-4103, ISSN: 0270-7306

### SEQUENCE LISTING

<110> Technische Universität Dresden
<120> Einrichtung und verfahren zum Nachweis einer Substanz mittels partikel-Plasmonen-Resonanz (PPR) oder Partikel-vermittelter Fluoreszenz auf der Basis von zelloberflächenpolarisierungen
<130> 17P0102DEWO
<160> 8
<170> PatentIn version 3.5
<210> 1
   <211> 36
   <212> DNA
   <213> Artificial Sequence
<220>
   <223> Primer
<400> 1
   tattatgagc tcggtgctgt gaccgtttcc aatacg 36
<210> 2
   <211> 44
   <212> DNA
   <213> Artificial sequence
<220>
   <223> Primer
<400> 2
   tattatacta gttggtattt ctgatgatgt tcttgctctc tttg 44
<210> 3
   <211> 40
   <212> DNA
   <213> Artificial Sequence
<220>
   <223> Primer
<400> 3
   tattatacta gtatgagatt tccttcaatt tttactgcag 40
<210> 4
   <211> 33
   <212> DNA
   <213> Artificial Sequence
<220>
   <223> Primer
<400> 4
   tattatgtcg acttagtaca ttggttggcc ggg 33
<210> 5
   <211> 70
   <212> DNA
   <213> Artificial Sequence
<220>
   <223> Primer
<400> 5
   aagaagatta caaactatca atttcataca caatataaac gattaaaaga cggatccccg 60
   ggttaattaa 70
<210> 6
   <211> 70
   <212> DNA
   <213> Artificial Sequence
<220>
   <223> Primer
<400> 6
   tgggaacaaa gtcgactttg ttacatctac actgttgtta tcagtcgggc gaattcgagc 60
   tcgtttaaac 70
<210> 7
   <211> 70
   <212> DNA
   <213> Artificial Sequence
<220>
   <223> Primer
<400> 7
   ttactaccat cacctgcatc aaattccagt aaattcacat attggagaaa cggatccccg 60
   ggttaattaa 70
<210> 8
   <211> 70
   <212> DNA
   <213> Artificial Sequence
<220>
   <223> Primer
<400> 8
   atgaacgtga aagaaatcga gagggtttag aagtagttta gggtcatttt gaattcgagc 60
   tcgtttaaac 70

## Beschreibung

Die Erfindung betrifft Einrichtungen und Verfahren zum Nachweis einer Substanz durch Zelloberflächenpolarisierung und deren Detektion mittels PPR oder Partikel-vermittelter Fluoreszenz.

E. Z. Ron beschreibt zellbasierte Biosensoren, um Umweltverschmutzung und Toxizität zu messen (Ron, E.Z. (2007) Biosensing environmental pollution. Current Opinion in Biotechnology 18:252-256). Zellen enthalten ein Reporter-Gen, wie zum Beispiel beta-Galaktosidase, FireflyLuciferase oder Green Fluorescent Protein, das unter die Kontrolle eines durch die nachzuweisende Substanz regulierbaren Promotors gestellt ist. Die Expression des Reporter-Gens wird durch Farbe, Biolumineszenz, Fluoreszenz oder elektrochemische Reaktion bestimmt.

Bisherige Systeme erlauben es nicht, von lebenden Zellen detektierte Signale so zu wandeln, dass sie sich mittels PPR oder Partikel-vermittelter Fluoreszenz in unmittelbarer Nachbarschaft der lebenden Zellen auf einfache Weise detektieren lassen.

Aufgabe der Erfindung ist es, eine Einrichtung und ein Verfahren zum Nachweis einer Substanz mittels eines biologischen Systems unter Nutzung von Partikel-Plasmonen-Resonanz (PPR) oder Partikel-vermittelter Fluoreszenz bereitzustellen, mit der Zielmoleküle ggf. auch in geringen Konzentrationen detektiert werden können.

Erfindungsgemäß wird die Aufgabe gelöst durch eine Einrichtung zum Nachweis einer Substanz durch Zelloberflächenpolarisierung mit den Merkmalen des Anspruchs 1, nämlich mit
a) Zellen, bei denen ein Gen, dessen Expression zu der polarisierten Präsentation eines Proteins auf der Oberfläche von Zellen führt, unter die Kontrolle eines durch die nachzuweisende Substanz regulierbaren Promotors gestellt ist,
b) Nanopartikeln, die mit einem Molekül funktionalisiert sind, das spezifisch an das Oberflächen-exponierte Protein binden kann, und
c) wenigstens einer optischen Messeinrichtung,
so dass durch Partikel-Plasmonen-Resonanz oder Partikel-vermittelte Fluoreszenz eine Zusammenlagerung der Nanopartikel auf der Oberfläche der Zellen nachgewiesen werden kann.

Ein typisches Beispiel der Polarisierung von Oberflächen ist die Ausbildung der immunologischen Synapse. Werden z.B. humane T-Zellen durch die Präsentation eines Antigens aktiviert, kommt es zu einer Umlagerung der vorher auf der Zelloberfläche ubiquitär verteilten T-Zell Rezeptoren zum Punkt der Antigen-Erkennung. Durch die Umlagerung kommt es zu einer starken Akkumulation der T-Zell Rezeptoren in einem begrenzten Areal der Zelloberfläche, der so genannten immunologischen Synapse (van der Merwe, P. A. et al. (2000) Cytoskeletal polarization and redistribution of cell-surface molecules during T-cell antigen recognition. Seminars in Immunology 12:5-21).

Ebenfalls zu Polarisierungseffekten bei T-Zellen führen die Aktivierungen von beispielsweise den CD4- und CTLA-4-Rezeptoren (Nguyen, D.H. et al. (2005) Dynamic reorganization of chemokine receptors, cholesterol, lipid rafts, and adhesion molecules to sites of CD4 engagement. Experimental Cellular Research 304:559-569; Wei, B. et al. (2007) CTLassociated antigen-4 ligation induces rapid T-cell polarization that depends on phosphatidylinositol 3-kinase, Vav-1, Cdc42, and myosin light chain kinase. The Journal of Immunology 179:400-408).

Ein weiteres Beispiel für eine hochgradige Polarisierung eines Biomoleküls an Zelloberflächen nach spezifischer Induktion zeigt *Dictyostelium discoideum.* Es handelt sich um einen einzelligen Schleimpilz, der als Amöbe oder nach Induktion als Zellaggregat bis zu einem vielzelligen Fruchtkörper hin organisiert vorkommen kann. Die Aggregation der Einzelzellen wird chemotaktisch gesteuert und erfordert ein hochgradig reguliertes genetisches Programm, welches letztlich zum Aufbau eines Fruchtkörpers führt. Wird *D. discoideum* einem chemischen Gradienten (z.B. cAMP) ausgesetzt, erfolgt eine Polarisierung der Zelloberfläche. Ein typischer Marker dieser Polarisierung ist das Phospholipid PIP3, welches an der Zelloberfläche zum Punkt der höchsten Konzentration des Gradienten hin spezifisch akkumuliert. Die Akkumulation von PIP3 wird in vielen chemotaktischen Zellen beobachtet (Franca-Koh, J. et al. (2006) Navigating signaling networks: chemotaxis in Dictyostelium discoideum. Current Opinion in Genetics and Development 16:333-338).

Ein weiteres Beispiel für ein Protein, das nach Induktion geclustert auf der Zelloberfläche auftritt, ist der Chemokinrezeptor CCR2. CCR2 ist ein membranständiger, G-Protein gekoppelter Rezeptor, der aus der Zelle herausragt. Er wird durch Chemokine aktiviert und überträgt das Signal über die Plasmamembran, was zur Aktivierung einer nachgeschalteten Signalkaskade führt. CCR2 wird als Antwort auf spezifische Reize z.B. in Lymphozyten oder auch in multipotenten adulten mesenchymalen Stammzellen polarisiert auf der Zelloberfläche präsentiert (Belema-Bedada, F. et al. (2008) Efficient homing of multipotent adult mesenchymal stem cells depends on FROUNT-mediated clustering of CCR2. Cell Stem Cell 2:566-575; Nieto, M. et al. (1997) Polarization of chemokine receptors to the leading edge during lymphocyte chemotaxis. Journal of Experimental Medicine 186:153-158).

In einer bevorzugten Ausgestaltung führt die durch die nachzuweisende Substanz induzierte Expression des Gens in einer ersten Zelle zu einer polarisierten Präsentation eines Proteins auf der Oberfläche von Zellen eines zweiten Zelltyps. Die entsprechende Ausgestaltung besitzt daher die Merkmale des Anspruchs 2, nämlich
- Zellen eines ersten Typs, bei denen ein für ein Pheromon kodierendes Gen unter die Kontrolle eines durch die nachzuweisende Substanz regulierbaren Promotors gestellt ist, so dass durch das Auftreten der nachzuweisenden Substanz die Zellen des ersten Typs das Pheromon sezernieren, und
- Zellen eines zweiten Typs, die für das Pheromon responsiv sind und deren Oberfläche durch das Auftreten des Pheromons polarisiert wird.

Die Einrichtung gemäß Anspruch 2 kann demnach folgendermaßen zusammengefasst werden als eine Einrichtung mit
- Zellen eines ersten Typs, bei denen ein für ein Pheromon kodierendes Gen unter die Kontrolle eines durch die nachzuweisende Substanz regulierbaren Promotors gestellt ist, so dass durch das Auftreten der nachzuweisenden Substanz die Zellen des ersten Typs das Pheromon sezernieren,
- Zellen eines zweiten Typs, die für das Pheromon responsiv sind und deren Oberfläche durch das Auftreten des Pheromons polarisiert wird,
- Nanopartikeln, die mit einem Antikörper funktionalisiert sind, der gegen ein Protein gerichtet ist, das durch das Auftreten des Pheromons auf der Oberfläche der Zellen des zweiten Typs polarisiert exponiert wird, und
- wenigstens einer optischen Messeinrichtung,
so dass durch Partikel-Plasmonen-Resonanz oder optische Fluoreszenz eine messbare Zusammenlagerung der Nanopartikel auf der Zelloberfläche der Hefezellen des zweiten Typs nachgewiesen werden kann.

In der erfindungsgemäßen Einrichtung befinden sich die Zellen in einem flüssigen, bevorzugt wässrigen Medium. Die Zellen der Einrichtung sind dabei entweder suspendiert in Lösung oder immobilisiert auf einem Träger.

Die Lösung befindet sich in einem geeigneten Behältnis, das die messtechnische Erfassung eines von den Zellen ausgesandten Signals gewährleistet.

Der Träger ist ebenfalls so gestaltet, dass von den Zellen ausgesandte Signale durch ein Detektionssystem erfasst werden können.

Die nachzuweisenden Substanzen befinden sich ebenfalls in wässriger Lösung. Für den Nachweis werden die zu testenden Lösungen mit den Zellen der erfindungsgemäßen Einrichtung kontaktiert.

Vorteilhafte Ausgestaltungen der erfindungsgemäßen Einrichtung sind in den Patentansprüchen 3 bis 13 angegeben.

Nach der Weiterbildung des **Patentanspruchs 3** sind die Zellen Hefezellen. In einer Ausführungsform sind die Zellen *Saccharomyces cerevisiae* oder *Schizosaccharomyces pombe* Hefezellen.

Bei Hefezellen unterscheidet man zwei so genannte Paarungstypen. Bei der Bäckerhefe *Saccharomyces cerevisiae* sind dies die Paarungstypen α und a, bei der Spalthefe *Schizosaccharomyces pombe* Plus und Minus.

Die Hefezellen des ersten Typs sind in einer Ausführungsform *Saccharomyces cerevisiae-Zellen* des α-Paarungstyps oder *Saccharomyces cerevisiae*-Zellen das α-Paarungstyps.

Die Hefezellen des zweiten Typs sind in einer Ausführungsform *Saccharomyces cerevisiae-Zellen* das α-Paarungstyps oder *Saccharomyces cerevisiae*-Zellen des α-Paarungstyps. Die Hefezellen des zweiten Typs verfügen über Rezeptoren sowie eine zugehörige intrazelluläre Signalkaskade zur Weiterleitung des Signals für das Pheromon, welches von den Zellen des ersten Typs gebildet wird.

Die jeweiligen Hefezellen bilden kurze Peptide, sog. Pheromone, um ihrer Umgebung den eigenen Paarungstyp mitzuteilen. So sezernieren z.B. *Saccharomyces cerevisiae-Zellen* des Paarungstyps α das Pheromon α-Faktor und Zellen des Paarungstyps a das Pheromon a-Faktor. Die Hefezellen besitzen auf ihren Oberflächen Rezeptoren für die Pheromone des jeweils entgegengesetzten Paarungstyps. So sind beispielsweise *Saccharomyces cerevisiae-*Zellen des Paarungstyps a in der Lage, *Saccharomyces cerevisiae-Zellen* des Paarungstyps α in ihrer Umgebung wahrzunehmen und umgekehrt.

Nehmen Hefezellen eines Paarungstyps Pheromone des entgegengesetzten Paarungstyps in ihrer Umgebung wahr, wird ein umfangreiches genetisches Programm gestartet, dessen Ziel die Verschmelzung jeweils einer Hefezelle eines Paarungstyps mit einer Hefezelle des entgegengesetzten Paarungstyps unter Bildung einer diploiden Zygote ist.

Erfindungsgemäß werden Hefezellen eines ersten Typs genetisch so verändert, dass ein Gen, das für ein Pheromon kodiert, unter die Kontrolle eines Promotors gestellt ist, der durch ein Signal reguliert wird.

Als Promotor wird in der Genetik eine DNA-Sequenz bezeichnet, die die Expression eines Gens reguliert. Promotoren im Sinne der Erfindung sind bevorzugt diejenigen Bereiche der genomischen DNA, die spezifisch für die Regulation der Expression eines Gens verantwortlich sind, indem sie auf spezifische intra- oder extrazelluläre Signale reagieren und abhängig von diesen Signalen die Expression des unter ihrer Kontrolle liegenden Gens aktivieren oder reprimieren. In Hefen befinden sich diese regulierenden DNA-Bereiche in der Regel auf der 5'-Seite des Startcodons des betreffenden Gens und haben eine durchschnittliche Länge von 309 bp (Mewes H. W. et al., Overview of the yeast genome. Nature (1997) 387, 7-65). Solche regulierenden Bereiche können aber auch weiter als 1000 bp entfernt von der kodierenden Sequenz oder auf der 3'-Seite der kodierenden Sequenz des betreffenden Gens oder sogar innerhalb der transkribierten Sequenz des betreffenden Gens liegen. Setzt man derartige Promotoren an die 5'-Seite des Startcodons eines beliebigen Gens, bevorzugt eines Pheromongens, regulieren sie die Aktivität dieses Gens in Abhängigkeit von den oben genannten spezifischen Signalen.

Das Gen, das für ein Pheromon kodiert, ist nach der Weiterbildung des **Patentanspruchs 4** das *MFα1*-Gen, das *MFα2*-Gen, das *MFA1*-Gen oder das *MFA2*-Gen.

Das unter die Kontrolle eines Promotors eines Gens, das durch ein spezifische Signal reguliert wird, gestellte Pheromongen wird in eine Hefezelle eingebracht. Dabei kann es in der Hefezelle auf einem extrachromosomalen DNA-Molekül vorliegen. Bevorzugt verwendet wird dazu ein Hefe-Expressionsvektor, der bei der Teilung der Hefezelle stabil repliziert wird. Besonders bevorzugt ist ein sogenannter "high copy number" Vektor, der in der Hefezelle in einer großen Anzahl an Kopien vorliegt. Alternativ werden als extrachromosomale DNA-Moleküle auch künstliche Hefechromosomen (yeast artificial chromosomes) verwendet.

In einer anderen Ausführungsform wird das Pheromongen zusammen mit dem Promotor in die chromosomale DNA der Hefezelle integriert. Dadurch wird vorteilhaft sichergestellt, dass alle Nachkommen der Hefezelle ebenfalls das Pheromongen unter Kontrolle des spezifischen Promotors enthalten.

Alternativ werden auch Vektoren genutzt, die in geringerer Kopienzahl oder als einzelner Vektor in Hefen vorliegen, z.B. *ARS-CEN*-Vektoren.

Erkennen die Hefezellen des ersten Typs mittels Rezeptoren eingehende Signale, wird direkt oder indirekt über zwischengeschaltete Signalkaskaden die Transkription des signalspezifischen Promotors induziert, so dass die Hefezellen des ersten Typs als Antwort auf das eingehende Signal das Pheromon in die Umgebung sezernieren.

Die Hefezellen des zweiten Typs besitzen auf ihrer Oberfläche Rezeptoren für die Pheromone, die durch die Hefezellen des ersten Typs sezerniert werden. Erreicht das sezernierte Pheromon die umliegenden Hefezellen des zweiten Typs, werden die Hefezellen des zweiten Typs unter dem Einfluss der Pheromone des ersten Typs in einer bestimmten Zellzyklusphase (G1) arretiert, Stoffwechselprozesse werden modifiziert und die Hefezellen des ersten und zweiten Typs wachsen gezielt aufeinander zu. Dieser Effekt wird bei *S*. *cerevisiae* auch als "shmoo-Phänotyp" bezeichnet. Mit diesem Zellwachstum in Richtung auf den Paarungspartner geht eine hochgradige Veränderung und Polarisierung der Zelloberfläche der Hefezellen einher. Insbesondere erfährt die Zellspitze der Hefezellen des zweiten Typs, die in Richtung der Hefezelle des ersten Typs bzw. der das Pheromon sezemierenden Zelle wächst, umfangreiche Modifikationen in ihrer Lipid- und Proteinzusammensetzung. So liegen hier hochspezifisch bestimmte Proteine in hoher Konzentration vor.

Nach der Weiterbildung des **Patentanspruchs 5** ist die Zelle eine haploide Hefezelle, die gentechnisch so verändert ist, dass das Gen für ein Pheromon des entgegengesetzten Paarungstyps unter die Kontrolle eines Promotors gestellt ist, der durch die nachzuweisende Substanz reguliert wird.

Die Zelle ist für diese sezernierten Pheromone responsiv. Dadurch führt die durch die nachzuweisende Substanz induzierte Expression des Gens in dieser Zelle zu einer polarisierten Präsentation eines Proteins auf der Oberfläche. Diese kann dann über die Anlagerung der funktionalisierten Nanopartikel mittels Partikel-Plasmonen-Resonanz oder optische Fluoreszenz nachgewiesen werden.

In den Hefezellen des ersten und/oder zweiten Typs ist in einer Ausführungsform die authentische Regulation der Expression von Pheromonen ausgeschaltet.

Beispielsweise sind die natürlichen Gene *MFα1* und *MFα2,* welche beide den α-Faktor kodieren, in α-Zellen von *Saccharomyces cerevisiae-zellen* in einer Ausführungsform deletiert. Damit wird vorteilhaft sichergestellt, dass der α-Faktor ausschließlich dann gebildet und sezerniert wird, wenn das zu detektierende Signal vorhanden ist.

Beispielsweise sind die natürlichen Gene *MFA1* und *MFA2* in α-Zellen von *Saccharomyces cerevisiae-* Zellen deletiert. Damit werden vorteilhaft sekundäre Effekte auf die α-Zellen oder α-Zellen ausgeschlossen.

Erfindungsgemäß sind Nanopartikel mit einem Molekül funktionalisiert, das spezifisch an ein Protein bindet, das durch das Pheromon des ersten Paarungstyps auf der Zelloberfläche der Hefezellen des zweiten Paarungstyps polarisiert exponiert wird. Nanopartikel sind Teilchen mit einem Durchmesser von ca. 1 nm bis ca. 500 nm, deren optische Eigenschaften sowohl von der Partikelgröße als auch von der Partikelform stark abhängen. Bei Nanopartikel mit Größen typischerweise > 3 nm bestimmen Plasmonenresonanzen das optische Verhalten, während bei Nanopartikeln mit einer Teilchengröße < 3 nm eine Partikel-vermittelte Fluoreszenz beobachtet wird. Agglomerationen der Nanoteilchen führen infolge elektromagnetischer Wechselwirkung zu einer Änderung der Plasmonenresonanzen (Frequenzverschiebung) bzw. einer Änderung des Fluoreszenzspektrums.

In der Weiterbildung des **Patentanspruchs 6** bestehen die Nanopartikel aus Gold, Silber oder einer Legierung dieser Metalle.

Bevorzugt werden als Moleküle, die spezifisch an das polarisiert auf der Oberfläche von Zellen des zweiten Typs präsentierte Proteine binden. Dabei handelt es sich z.B. um Peptidliganden, Rezeptoren, Antikörper, Haptene oder um Nukleinsäuren. Bevorzugt binden diese Moleküle an den Bereich des Proteins, der an der Außenseite der Zelloberfläche zugänglich ist.

In Anwesenheit der Nanopartikel, z.B. Gold-Nanopartikel, die mit Molekülen funktionalisiert sind, die spezifisch an ein Protein binden, das durch das Pheromon auf der Zelloberfläche polarisiert präsentiert wird, kommt es zur Clusterbildung an den entsprechenden Oberflächenarealen der Hefezellen. Die daraus resultierende Frequenz - bzw. Farbänderung aufgrund der Partikel-Plasmonen-Resonanz bzw. der Partikel-vermittelten Fluoreszenz wird sensortechnisch erfasst. Durch Partikel-Plasmonen-Resonanz oder Partikel-vermittelte Fluoreszenz kann dadurch erfindungsgemäß eine messbare Zusammenlagerung der Nanopartikel auf der Zelloberfläche der Zellen des zweiten Paarungstyps nachgewiesen werden. Eine zunehmende Aggregation der Nanopartikel (Ausbildung von Clustern auf der Zelloberfläche) spiegelt sich in einer Frequenzänderung wider (Rotverschiebung).

Die erfindungsgemäße Einrichtung hat den Vorteil, dass ein von einer Zelle aufgenommenes Signal gewandelt und schließlich mittels Partikel-Plasmonen-Resonanz oder Partikel-vermittelter Fluoreszenz nachgewiesen werden kann. Das durch eine Zelle aufgenommene Signal kann dabei ggf. verstärkt werden.

In einer Ausführungsform besitzen die Nanopartikel einen Durchmesser von größer 3 nm, deren Anlagerung an Zellen des zweiten Typs mittels Partikel-Plasmonen-Resonanz nachgewiesen werden kann.

Die Nanopartikel besitzen in einer Ausführungsform einen Durchmesser von kleiner 3 nm, deren Anlagerung an Zellen des zweiten Paarungstyps mittels Partikel-vermittelter Fluoreszenz nachgewiesen werden kann.

Die Anbindung der spezifisch bindenden Moleküle an die Nanopartikel, die einen Durchmesser von größer als 3 nm besitzen, erfolgt sowohl direkt mittels unspezifischer Adsorption der Moleküle auf der Oberfläche der Nanopartikel oder aber gerichtet spezifisch im Falle einer vorausgegangen Funktionalisierung der Nanopartikeloberfläche (bspw. durch chemisch funktionelle endständige Gruppen).

Nanopartikel, die einen Durchmesser von kleiner 3 nm besitzen, werden an Antikörper als spezifisch bindende Moleküle gebunden, und können des Weiteren mittels Antikörper-gekoppelter amphiphiler Kern-Schalestrukturen auf der Basis saccharidfunktionalisierter dendritischer Polymere funktionalisiert werden.

Um die Zugänglichkeit der Zelloberfläche für die funktionalisierten Nanopartikel zu erleichtern, wird eine zellwandlose Hefemutante eingesetzt oder die Zellwand durch vorhergehenden enzymatischen Abbau entfernt. Hierzu müssen die Zellen vorher in eine osmostabilisierende Matrix (z.B. 1% Agarose) eingebettet oder in einem osmotisch stabilisierenden Medium (z.B. in 1 M Sorbitol) gehalten werden, um die Integrität der Zellen zu gewährleisten.

Das Protein, das durch das Pheromon auf der Zelloberfläche polarisiert präsentiert wird und an das das spezifische Molekül, mit dem die Nanopartikel funktionalisiert sind, bindet, ist nach der Weiterführung des **Patentanspruchs** 7 Fus1p.

Fus1p aus *Saccharomyces cerevisiae* ist für die Fusion der Zellen notwendig und liegt deshalb in den aufeinander zuwachsenden Zellspitzen in hoher Konzentration vor. Es handelt sich um ein Protein, das die Cytoplasmamembran mit einer Transmembrandomäne durchspannt. Der N-Terminus von Fus1p ist nach außen und der größere C-terminale Bereich ins Zellinnere gerichtet. Damit ist der N-terminale Bereich von Fus1p als Antwort der Zelle auf das Einwirken des jeweiligen entgegen gesetzten Pheromons spezifisch an der Zelloberfläche der aufeinander zu wachsenden Zellen konzentriert.

Bevorzugte, spezifisch an Fus1p bindende Moleküle sind dabei anti-Fus1p-Antikörper. Als Antikörper im erfindungsgemäßen Sinn sind weiterhin verschiedene modifizierte Formen von Antikörpern zu verstehen, wie z.B. Fragmente wie das Fv-Fragment, das Fab-Fragment oder das (Fab)'2-Fragment.

Neben dem angegebenen Protein werden auch andere Proteine nur partiell bzw. polarisiert auf der Zelloberfläche präsentiert. Auch solche Proteine können genutzt werden. Außerdem wird auch die Zusammensetzung der Zellwand an den "Zellspitzen" modifiziert und kann somit für das beschriebene Verfahren genutzt werden.

Weiterhin werden bevorzugt Proteine eingesetzt, die durch die Aktivierung des Pheromonsignalwegs in Zellen des zweiten Typs polarisiert auf der Zelloberfläche exponiert werden, und die in dem Bereich, der von außerhalb der Zelle zugänglich ist, mit einem Epitop-Tag verbunden sind. Solche Epitop-Tags sind kurze Moleküle, meist Oligopeptide, die auch multimerisiert vorliegen können, und die von Antikörpern spezifisch gebunden werden. Bevorzugte Epitop-Tags sind solche, die die Präsentation des an den Epitop-Tag gebundenen Proteins nicht behindern oder negativ beeinflussen. Besonders bevorzugt sind HA-Tag, Myc-Tag, Flag-Tag, SUMO-Tag, His-Tag oder der T7-Tag. Bevorzugt können auch Proteine (z.B. eGFP) oder Proteindomänen in einem Fusionsprotein mit dem polarisiert auf der Oberfläche präsentierten Protein eingesetzt werden, die von Antikörpern oder Nukleinsäuren spezifisch gebunden werden. Als Moleküle, die spezifisch an die polarisiert auf der Oberfläche der Zellen des zweiten Typs präsentierten Proteine binden, werden spezifisch gegen den verwendeten Epitop-Tag oder das fusionierte Protein bzw. die Proteindomäne gerichtete Antikörper oder an einen DNA-bindenden Proteinteil bindende Nukleinsäuren verwendet. Dadurch wird vorteilhaft eine zuverlässige und hochspezifische Bindung der Nanopartikel an die polarisiert präsentierten Proteine gewährleistet, auch wenn ein spezifisch gegen dieses Protein selbst gerichteter Antikörper nicht zur Verfügung steht.

Nach der Weiterbildung des **Patentanspruchs 8** ergibt sich durch eine geeignete Wahl des Verhältnisses von Zellen des ersten Typs zu Zellen des zweiten Typs eine Signalverstärkung. Dabei kann durch die gezielte Beeinflussung der Zahlenverhältnisse der verschiedenen Zelltypen der verstärkende Effekt weiter gesteigert werden. So liegen dabei die Zellen des ersten Typs gegenüber den Zellen des zweiten Typs in einem Verhältnis von 1 zu 20, bevorzugt von 1 zu 10, besonders bevorzugt von 1 zu 5 vor.

Die Zellen der erfindungsgemäßen Einrichtung können suspendiert in Lösung oder immobilisiert sein. Nach der Weiterbildung des **Patentanspruchs 9** befinden sich die Zellen in einem porösen organischen oder anorganischen Gel, in einer weiteren Ausführungsform in einem porösen und optisch transparenten Siliziumdioxid-Xerogel.

Xerogele sind Gele, die ihre Flüssigkeit beispielsweise durch Verdampfen oder Absaugen verloren haben. Gele sind formbeständige, leicht deformierbare disperse Systeme aus mindestens zwei Komponenten, die zumeist aus einem festen Stoff mit langen oder stark verzweigten Teilchen (z.B. Kieselsäure, Gelatine, Kollagene, Polysaccharide, Pektine, spezielle Polymere, wie z.B. Polyacrylate, und andere, oft als Verdickungsmittel bezeichnete Geliermittel) und einer Flüssigkeit (meist Wasser) als Dispersionsmittel bestehen. Dabei bildet die feste Substanz im Dispersionsmittel ein räumliches Netzwerk. Bei der Entstehung von Xerogelen verändert sich die räumliche Anordnung des Netzes.

Die erfindungsgemäße Verwendung von anorganischen oder biologisch inerten organischen Xerogelen zur Einbettung der Zellen erlaubt vorteilhaft das Überleben der Zellen bei gleichzeitiger Stabilität der erzeugten Strukturen, denn sie sind toxikologisch und biologisch inert und werden im Allgemeinen nicht durch die Hefen abgebaut. Sie ermöglichen weiterhin vorteilhaft die Einlagerung von Nährstoffen und Feuchthaltemitteln, die das Überleben der Zellen sichern.

Die Zellen sind erfindungsgemäß in einem porösen und optisch transparenten anorganischen oder biologisch inerten organischen Xerogel immobilisiert. Bevorzugt ist das Xerogel ein anorganisches Xerogel aus Siliziumdioxid, alkyliertem Siliziumdioxid, Titandioxid, Aluminiumoxid oder deren Gemischen, und wird vorzugsweise durch einen Sol-Gel-Prozess hergestellt.

Dazu werden zunächst Silica- oder andere anorganische Nanosole entweder durch säure- oder alkalikatalysierte Hydrolyse der entsprechenden Silizium- oder Metallalkoxide in Wasser oder einem wasserlöslichem organischem Lösungsmittel (wie Ethanol) hergestellt. Bevorzugt wird die Hydrolyse in Wasser durchgeführt, um toxische Effekte des Lösungsmittels auf die einzubettenden Zellen zu verhindern. Bei der Herstellung von Nanosolen durch Alkoxidhydrolyse entstehen im Zuge der Reaktion Alkohole, die anschließend aus dem erhaltenen Nanosol durch Durchleitung eines inerten Gasstroms verdampft und durch Wasser ersetzt werden.

Durch die Verwendung von Gemischen verschiedener Alkoxide können die Matrixeigenschaften gezielt beeinflusst werden. Die Sol-Gel-Matrix erlaubt vorteilhaft die chemische Modifizierung durch Co-Hydrolyse und Co-Kondensation unter Verwendung verschiedener Metalloxide von Metallen wie Al, Ti, Zr zur Herstellung von gemischten Oxiden oder von Alkoxysilanen mit organischen Resten am Si-Atom zur Herstellung von organisch modifizierten Siliziumoxid-Gelen.

Die einzubettenden Zellen werden mit dem entstandenen Nanosol gemischt. Der Prozess der Gelbildung wird bevorzugt eingeleitet durch Erhöhung der Temperatur, Neutralisierung des pH-Werts, Aufkonzentrierung oder die Zugabe von Katalysatoren wie beispielsweise Fluoriden. Dabei sollte die Temperatur jedoch nicht auf Temperaturen >42°C erhöht werden, um die einzubettenden Zellen nicht zu schädigen. Bei der Überführung in ein Gel verringern die Nanosole ihr Oberflächen/Volumenverhältnis durch Aggregation und dreidimensionale Quervernetzungen. Während dieser Umwandlung des Nanosols in ein sogenanntes Lyogel werden die Zellen in dem entstehenden anorganischen Netzwerk immobilisiert. Die Immobilisierung überlebensfähiger Zellen wird vorteilhaft durch das Verhältnis Zellen:Oxid und durch die Zugabe von Poren-formenden Agentien gesteuert.

Der Anteil von Zellen an der Gesamtmenge des erzeugten Xerogels einschließlich der eingebetteten Zellen kann je nach Anwendung von 0,1 bis 50% Gewichtsprozent betragen. Bevorzugt verwendet wird ein Anteil von 2 bis 25% Gewichtsprozent.

Durch Trocknung wird dem Lyogel das noch enthaltene Lösungsmittel entzogen. Dadurch bildet sich aus dem Lyogel das Xerogel. Das entstehende Xerogel weist eine hohe Porosität auf, die einen raschen Stoffaustausch mit dem umgebenden Medium erlaubt. Der Trocknungsprozess hat eine starke Schrumpfung des Gels zur Folge, der zu Stress für die eingebetteten Zellen führt. Bevorzugt wird der Trocknungsschritt daher sehr schonend und langsam bei Temperaturen von weniger als 40°C durchgeführt.

Mit sinkendem Wassergehalt der Matrix verringern sich die physiologische Aktivität und die Überlebensrate der eingebetteten Zellen. Ein zu hoher Wassergehalt führt jedoch zu niedriger mechanischer Stabilität und verringert die Haltbarkeit der Struktur.

Die erfindungsgemäße Verwendung von Hefezellen ist daher besonders vorteilhaft, da Hefezellen eine hohe Resistenz gegen Trockenheit besitzen und auch bei sehr geringem Wassergehalt ihre Überlebensfähigkeit nicht einbüßen. Dadurch wird es möglich, sehr trockene Xerogele herzustellen.

Die Erfindung umfasst auch die Verwendung verschiedener Additive wie lösliche organische Salze, d. h. Metallsalze organischer Carbon- oder Sulfonsäuren bzw. offenkettige oder cyclische Ammoniumsalze und Quartärsalze von N-Heterocyclen sowie niedermolekulare Polyanionen oder Polykationen, oder wasserlösliche organische Verbindungen wie Polycarbonsäuren, Harnstoff-Derivate, Kohlenhydrate, Polyole, wie Glycerin, Polyethylenglycol und Polyvinylalkohol, oder Gelatine, die als Weichmacher, Feuchthaltemittel und Porenbildner wirken, die Zelllyse hemmen und die Überlebensfähigkeit der eingebetteten Zellen beträchtlich verlängern.

Das Siliziumdioxid-Xerogel mit den Zellen befindet sich in einer Ausführungsform auf einem Substrat mit erhöhter mechanischer Stabilität.

Substrate sind in einer Ausführungsform eine Lichtleitfaser, Glasbeads, ein planarer Glasträger oder andere Formkörper aus Glas wie Hohlkugeln, Stäbe, Röhren oder keramische Granulate.

Dabei werden die Zellen in einem porösen und optisch transparenten anorganischen Xerogel, z. B. einem Siliziumdioxid-Xerogel, fixiert. Das mit den Mikroorganismen versetzte Siliziumdioxid-Xerogel wird als Schicht auf Glasbeads, einer Lichtleitfaser, planaren Glasträgern oder anderen Formkörpern wie Hohlkugeln, Stäben, Röhren oder keramischen Granulaten mittels eines bekannten Sol-Gel-Prozesses abgeschieden, indem das Nanosol-Zell-Gemisch auf das zu beschichtende Substrat aufgebracht oder das Substrat in das Nanosol-Zell-Gemisch eingetaucht wird und das Nanosol anschließend durch Trocknung und die dadurch resultierende Aufkonzentrierung des Nanosols in ein Xerogel überführt wird. Die dadurch vorhandene mechanische Stabilität dieser Strukturen erlaubt das Einbringen der erfindungsgemäßen Einrichtung in ein Messsystem, das unmittelbar mit dem zu untersuchenden Reaktionsraum (Fermenter) im Sinn einer near-line-Diagnostik verbunden werden kann.

in einer Ausführungsform sind die Zellen ein Bestandteil einer einen Hohlraum wenigstens teilweise umschließenden Hüllenstruktur. Das heißt, dass einzelne oder mehrere Zellen in diesem Hohlraum, der eine poröse Hülle hat, eingekapselt werden. Die Mikroporosität erlaubt vorteilhaft einen Stoffaustausch mit der Umgebung.

Die Hüllenstruktur besteht in einer Ausführungsform aus einem Grundkörper mit einer inneren Schicht aus einem biologischen Hydrogel und einer äußeren Schicht aus einem porösen anorganischen Gel, wobei die Schichten wenigstens bereichsweise aufgebracht sind.

Nach der Weiterbildung des **Patentanspruchs 10** sind die Zellen in ein Gefüge mit einer hierarchischen Porenstruktur eingebettet, so dass neben der für anorganische Gele typischen Nanoporosität das Gefüge zusätzlich von miteinander verbundenen Mesoporen durchzogen wird, deren Durchmesser typischerweise zwischen 10 bis 100 µm variiert und die einen Stoffaustausch zwischen der Umgebung und den eingebetteten Zellen sowie deren Reaktionsprodukten wie den Enzymen ermöglichen. Diese Mesoporen dienen zugleich als Transportwege für die als physikalische Sensoren dienenden Nanoteilchen.

Die Zellen befinden sich nach der Weiterbildung des **Patentanspruchs 11** wenigstens auf einer Oberfläche in einer transparenten Messzelle, wobei die Messzelle Einrichtungen zum Zuführen und Abführen eines Mediums besitzt und mit einer Heizeinrichtung gekoppelt ist.

Die Zellen sind in eine Ausführungsform ein Bestandteil einer Lösung oder eines Gels, die oder das sich in einem Behältnis als Messzelle befindet.

Die Messzelle ist Bestandteil einer optischen Messeinrichtung, die weiterhin aus wenigstens einer Quelle für elektromagnetische Strahlen und entweder einem Bildaufnahmesystem oder einem Fotodetektor besteht. Das Behältnis und die Lösung oder das Gel bestehen aus Materialien, die für die elektromagnetischen Strahlen der Quelle transparent sind.

Ein Bildaufnahmesystem ist nach der Weiterbildung des **Patentanspruchs 12** als optische Messeinrichtung als eine die Zellen abbildende Optik so angeordnet, dass eine durch die Zusammenlagerung der Nanopartikel hervorgerufene Farb-oder Resonanzfrequenz änderung von Zellen als Abbildungssignal quantitativ oder qualitativ bestimmbar ist.

Nach der Weiterbildung des **Patentanspruchs 13** sind eine Quelle für elektromagnetische Strahlen, Zellen und mindestens ein Fotodetektor als optische Messeinrichtung so angeordnet, dass elektromagnetische Strahlen der Quelle auf Zellen fallen und das dadurch hervorgerufene Fluoreszenzlicht als Abbildungssignal des Fotodetektors quantitativ oder qualitativ bestimmbar ist.

In einer Ausführungsform sind eine Quelle für elektromagnetische Strahlen, Zellen sowie Nanopartikel und wenigstens ein Fotodetektor als optische Messeinrichtung so angeordnet, dass durch die elektromagnetischen Strahlen der Quelle in den Nanopartikeln angeregte elektromagnetische Strahlen auf den Fotodetektor gelangen, abgebildet werden und als Abbildungssignale quantitativ oder qualitativ bestimmbar sind.

In einer Ausführungsform ist im Strahlengang nach der Quelle für elektromagnetische Strahlen und/oder im Strahlengang vor dem Fotodetektor wenigstens eine Strahlen beeinflussende Vorrichtung, wenigstens eine Strahlen formende Vorrichtung oder wenigstens eine Kombination daraus angeordnet.

In einer Ausführungsform ist der Fotodetektor ein Festkörperbildsensor mit Fotowiderständen, Fotodioden oder Fototransistoren, und der Festkörperbildsensor ist mit einem Datenverarbeitungssystem zusammengeschaltet.

Bestandteil der Erfindung ist nach **Patentanspruch 14** ein Verfahren zum Nachweis einer Substanz mittels Partikel-Plasmonen-Resonanz (PPR) oder Partikel-vermittelter Fluoreszenz durch Zelloberflächenpolarisierung unter Nutzung von Zellen, Nanopartikeln und wenigstens einer Messeinrichtung. Das Verfahren umfasst die folgenden Verfahrensschritte:
a) die Oberfläche von Zellen; in denen ein Gen, dessen Expression zu der polarisierten Präsentation eines Proteins auf der Oberfläche von Zellen führt, unter die Kontrolle eines durch die nachzuweisende Substanz regulierbaren Promotors gestellt ist, wird durch das Auftreten der Substanz polarisiert,
b) Nanopartikel, die mit einem Molekül funktionalisiert sind, das spezifisch an das Protein binden kann, das durch das Auftreten der Substanz auf der Oberfläche der Zellen polarisiert exponiert wird, binden an das Protein und
c) durch Partikel-Plasmonen-Resonanz oder Partikel-vermittelte Fluoreszenz wird mittels wenigstens einer optischen Messeinrichtung eine messbare Zusammenlagerung der Nanopartikel auf der Zelloberfläche der Zellen nachgewiesen.

Bestandteil der Erfindung ist nach **Patentanspruch 15** auch ein Verfahren, zum Nachweis einer Substanz mittels Partikel-Plasmonen-Resonanz (PPR) oder Partikel-vermittelte Fluoreszenz durch Zelloberflächenpolarisierung, wobei als Zellen Zellen eines ersten Typs und Zellen eines zweiten Typs genutzt werden, wobei
a) Zellen des ersten Typs, in denen ein für ein Pheromon kodierendes Gen unter die Kontrolle eines durch die nachzuweisende Substanz regulierbaren Promotors gestellt ist, durch das Auftreten der Substanz das Pheromon sezernieren, und
b) die Oberfläche von Zellen des zweiten Typs durch das Auftreten des Pheromons polarisiert wird.

Das Verfahren gemäß Anspruch 15 kann also durch folgende Verfahrensschritte zusammengefasst werden:
a) die Zellen des ersten Typs, in denen ein für ein Pheromon kodierendes Gen unter die Kontrolle eines durch die nachzuweisende Substanz regulierbaren Promotors gestellt ist, sezernieren auf die Anwesenheit der Substanz hin das Pheromon,
b) die Oberfläche von Zellen eines zweiten Typs, die für das Pheromon responsiv sind, wird durch das Auftreten des Pheromons polarisiert,
c) Nanopartikel, die mit einem Molekül funktionalisiert sind, das spezifisch an ein Protein binden kann, das durch das Auftreten des Pheromons auf der Oberfläche der Zellen des zweiten Typs polarisiert exponiert wird, binden an das Protein, und
d) durch Partikel-Plasmonen-Resonanz oder optische Fluoreszenz wird mittels wenigstens einer optischen Messeinrichtung eine messbare Zusammenlagerung der Nanopartikel auf der Oberfläche der Zellen des zweiten Typs nachgewiesen.

Für vorteilhafte Ausgestaltungen des erfindungsgemäßen Verfahrens, betreffend die Zellen, die Nanopartikel und die Messeinrichtung, gelten die in der Beschreibung der erfindungsgemäßen Einrichtung aufgeführten vorteilhaften Ausgestaltungen der Merkmale der Einrichtung gleichermaßen. In einer Ausführungsform, wird das Verfahren mit wenigstens einer Einrichtung mit mindestens einem Merkmal aus einem der Ansprüche 3 bis 13 durchgeführt.

Anhand folgender Figuren und Ausführungsbeispiele wird die Erfindung näher erläutert.

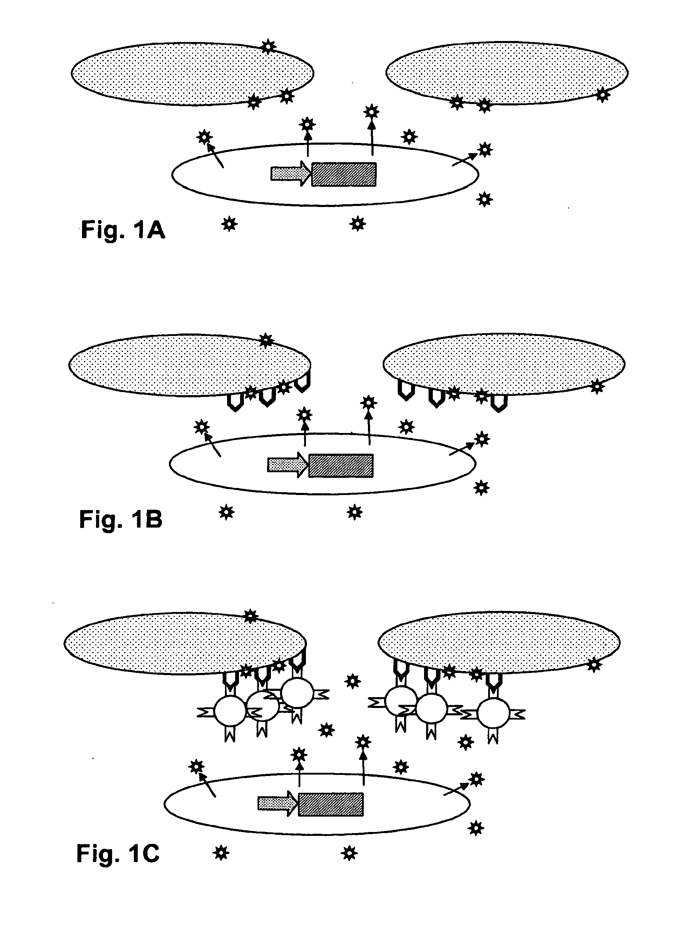

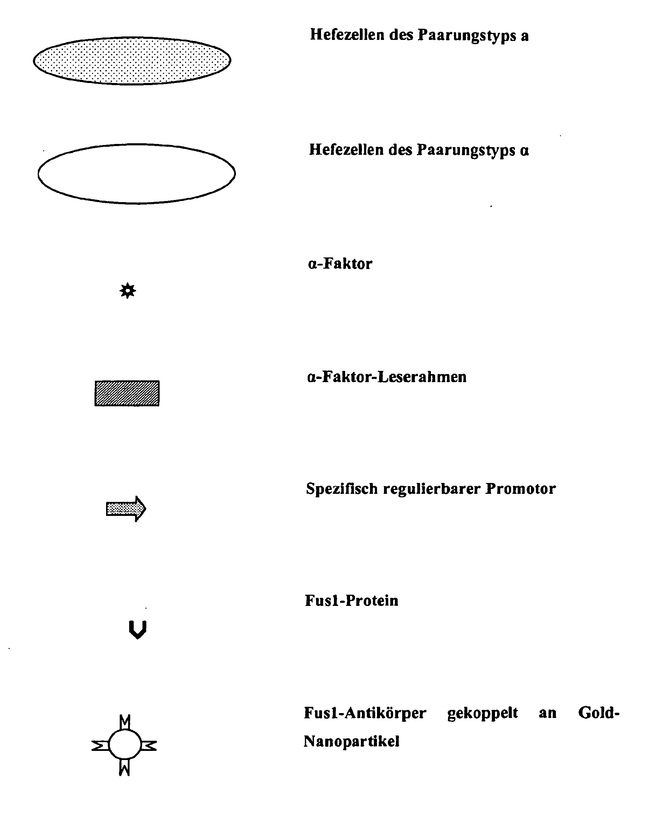

Dabei zeigen
- **Fig.1A:**: Schematische Darstellung von gentechnisch veränderten *Saccharomyces cerevisiae*-Hefezellen des Paarungstyps α und von *Saccharomyces cerevisiae-*Hefezellen des Paarungstyps a gemäß Ausführungsbeispiel 1.
- **Fig.1B:**: Schematische Darstellung der Polarisierung der Zelloberfläche und der Clusterung des Fus1p-Proteins an den Zellspitzen der Hefezellen des Paarungstyps a gemäß Ausführungsbeispiel 1.
- **Fig.1C:**: Schematische Darstellung der Goldclusterbildung an den entsprechenden Oberflächenarealen der Hefezellen des Paarungstyps a in Anwesenheit von Goldnanopartikeln, welche mit gegen den N-terminalen Bereich von Fus1 p oder eines gleichsinnig orientierten Fus1 p-Fusionsproteins gerichteten Antikörpern funktionalisiert sind gemäß Ausführungsbeispiel 1.

### Ausführungsbeispiel 1

*Saccharomyces cerevisiae*-Hefezellen des Paarungstyps α erkennen als Zellen des ersten Typs mittels eines Rezeptors ein eingehendes Signal. Rezeptoren induzieren direkt oder über zwischengeschaltete Signalkaskaden die Transkription des Promotors. Unter die Kontrolle des Promotors ist der für den α-Faktor kodierende *MFα1* Leserahmen kloniert, so dass die Hefezelle des Paarungstyps α als Antwort auf ein eingehendes Signal das Pheromon α-Faktor in die Umgebung sezerniert.

Die Herstellung einer solchen Hefezelle des ersten Typs ist im Folgenden beispielhaft für die Anwendung zum Monitoring von bioverfügbarem Phosphor beschrieben. Die Hefezellen des ersten Typs (Sensorzellen) reagiert hierbei sensitiv auf eine Limitierung von Phosphor. Das Gen *YAR071W* wird bei einer Phosphorlimitierung spezifisch sehr viel stärker transkribiert (Boer et al., (2003). The genome-wide transcriptional responses of Saccharomyces cerevisiae grown on glucose in aerobic chemostat cultures limited for carbon, nitrogen, phosphorus, or sulfur. J. Biol. Chem. 278: 3265-3274.). Die 1000 Basenpaare umfassende, stromaufwärtsliegende Region des heraufregulierten Gens *YAR071W* wird mittels der spezifischen Primer Seq.-Nr. 1 und Seq.-Nr. 2 aus Tab. 1 in einer PCR aus genomischer DNA von *Saccharomyces cerevisiae* amplifiziert. Durch die Primer wird die Sequenz um eine 5'-seitige Erkennungssequenz für *Sac*I und 3'-seitig um eine Erkennungssequenz für *Spe*I erweitert. Mittels dieser Erkennungssequenzen erfolgt ein gerichteter Einbau in den "high copy-number"-Vektor p426, folgend p426YAR071W genannt. Im zweiten Schritt wird der Leserahmen des *MFα1*-Gens in das Plasmid p426YAR071W kloniert. Dazu wird die Sequenz des *MFα1*-Leserahmens mit den Primem Seq.-Nr. 3 und Seq.-Nr. 4 (siehe Tab. 1) aus genomischer DNA von *Saccharomyces cerevisiae* amplifiziert, welche das Fragment 5'-seitig um eine *Spe*I*-* und 3'-seitig um eine *Sal*I-Schnittstelle erweitern. Danach erfolgt die Klonierung des Fragments mit den genannten Restriktionsschnittstellen in den Vektor p426YAR071W, folgend p426YAR071W-MFalpha1 genannt. Die korrekte Sequenz der klonierten Fragmente wird mittels DNA-Sequenzanalyse überprüft und bestätigt. Der Vektor p426 enthält einen URA3-Marker aus *Saccharomyces cerevisiae* zur Selektion in Uracil-auxotrophen Stämmen. Das entstandene Konstrukt p426YAR071W-MFalpha1 wird z.B. in den Hefestamm BY4742 (*MATα*, *his3*Δ*1*, *leu2*Δ*0*, *lys2*Δ*0, ura3*Δ*0*) transformiert und positive Transformanden selektiert. Bei Phosphorlimitierung wird in Sensorzellen, die mit dem Plasmid p426YAR071 W-MFalpha1 ausgestattet sind, spezifisch die Expression von α-Faktor induziert.

**Tab. 1: Primer für die Herstellung des Sensor-Plasmids p426YAR071W-MFalpha1. Zur genomischen Zielsequenz homologe Bereiche sind dick markiert, Erkennungssequenzen für Restriktionsendonukleasen sind unterstrichen.**

| **Nr.** | **Bezeichnung** | **Sequenz (5' → 3')** |
|---|---|---|
| 1 | YAR071W-for-SacI | TATTATGAGCTC**GGTGCTGTGACCGTTTCCAATACG** |
| 2 | YAR071W-rev-SpeI | TATTATACTAGT**TGGTATTTCTGATGATGTTCTTGCTCTCTTTG** |
| 3 | MFalpha1-for-SpeI | TATTATACTAG**TATGAGATTTCCTTCAATTTTTACTGCAG** |
| 4 | MFalpha1-rev-SalI | TATTATACTAGT**ATAGTACATTGGTTGGCCGGG** |

Die authentisch für den α-Faktor kodierenden Gene *MFα1* und *MFα2* sind im gleichen Stamm deletiert. Damit ist sichergestellt, dass der α-Faktor ausschließlich dann gebildet und sezerniert wird, wenn das zu detektierende Signal vorhanden ist.

Für die Deletion der Leserahmen von *MFα1* und *MFα2,* z.B. im α-Hefestamm BY4742 *(MATα, his3Δ1, leu2Δ0, lys2Δ0, ura3*Δ*0*), werden die Markerkassetten *natMX6* bzw. *hphMX6* verwendet, welche Resistenzen gegen die Antibiotika Nourseothricin bzw. Hygromycin B vermitteln. Die *natMX6*-Kassette wird in einer SFH-PCR mittels der Primer Seq.-Nr. 5 und Seq.-Nr. 6 aus Tab. 2 amplifiziert. Die 5'-Bereiche der Primer (je 50 Basen) sind homolog zu den flankierenden Sequenzen des *MFα1*-Leserahmens im Genom von *Saccharomyces cerevisiae.* Die 3'-Bereiche der Primer (20 bp) sind homolog zu den Enden der *natMX6-*Kassette. Als DNA-Template für die SFH-PCR dient das Plasmid pFA6a-natMX6. Anschließend werden Hefezellen mit dem SFH-Fragment transformiert. Transformanden, bei denen das Fragment über doppelt homologe Rekombination stabil in das Genom integriert ist, werden auf Nourseothricin-haltigem Medium selektiert und die korrekte Integration der Deletionskassette mittels diagnostischer PCR bestätigt. Danach erfolgt die Deletion des Leserahmens von *MFα2* im erzeugten *Δmfa1*-Hefestamm. Hierzu wird analog zur ersten Deletion ein SFH-Fragment mit den Primem Seq.-Nr. 7 und Seq.-Nr. 8 (siehe Tab. 2) und der hphMX6-Kassette (DNA-Template pFA6a-hphMX6) amplifiziert und in Δ*mfa1-*Hefezellen transformiert. Die 5'-seitigen Bereiche der Primer sind homolog zu den flankierenden Sequenzen des *MFα2*-Leserahmens im Genom von *Saccharomyces cerevisiae.* Die Selektion positiver Transformanden erfolgt auf Hygromycin B-haltigem Medium und die korrekte Integration der Hygromycin-Resistenzkassette im Δ*mfα1*-Δ*mfα2*-Hefestamm wird mittels diagnostischer PCR überprüft.

**Tab. 2: Primer für die Deletion der Leserahmen von MFα1 und MFα2 von Saccharomyces cerevisiae. Die unmarkierte Primersequenz kennzeichnet Bereiche, die homolog zur genomischen DNA von Saccharomyces cerevisiae sind, Zur Deletionskassette homologe Bereiche sind dick markiert.**

| **Seq.- Nr.** | **Bezeichnung** | **Sequenz (5' → 3')** |
|---|---|---|
| 5 | MFalp1_F2 | |
| 6 | MFalp1_R1 | |
| 7 | MFalp2_F2 | |
| 8 | MFalp2_R1 | |

Erreicht der sezernierte α-Faktor die umliegenden α-Zellen, erfolgt eine hochgradige Polarisierung der Zelloberfläche und die Clusterung des Fus1p Proteins an den Zellspitzen der a-Zellen (Fig. 1B). Diese Clusterung ist Voraussetzung für die Wandlung in ein PPR -Signal. Das durch die Expression und Sekretion des α-Faktors und die nachfolgende Polarisierung der Zellen hervorgerufene Signal kann durch das Verhältnis von α-Zellen zu α-Zellen moduliert werden.

In Anwesenheit von Goldnanopartikeln, welche mit gegen den N-terminalen Bereich von Fus1p oder eines gleichsinnig orientierten Fus1p-Fusionsproteins gerichteten Antikörpern funktionalisiert sind, kommt es zur Goldclusterbildung an den entsprechenden Oberflächenarealen der α-Zellen (Fig. 1C). Die daraus resultierende Farbänderung aufgrund der Partikel-Plasmonen-Resonanz oder Partikel-vermittelte Fluoreszenz wird sensortechnisch erfasst.

Die Quelle, die Messzelle und entweder das Bildaufnahmesystem oder der Fotodetektor sind so angeordnet, dass eine durch die elektromagnetischen Strahlen der Quelle hervorgerufene optische Änderung in der Messzelle auf dem Bildaufnahmesystem oder dem Fotodetektor abgebildet werden. Die Änderung ist beispielsweise
- eine durch die Zusammenlagerung der Nanopartikel hervorgerufene Farbänderung von Hefezellen oder
- ein durch die elektromagnetischen Strahlen der Quelle hervorgerufenes Fluoreszenzlicht der Hefezellen.

Die dadurch hervorgerufenen Abbildungssignale des Bildaufnahmesystems oder des Fotodetektors sind quantitativ oder qualitativ bestimmbar. Dazu ist das Bildaufnahmesystem oder der Fotodetektor mit einem Datenverarbeitungssystem zusammengeschaltet.

In Ausführungsformen können im Strahlengang nach der Quelle für elektromagnetische Strahlen und/oder im Strahlengang vor dem Fotodetektor wenigstens eine Strahlen beeinflussende Vorrichtung, wenigstens eine Strahlen formende Vorrichtung oder wenigstens eine Kombination daraus angeordnet sein. Strahlen formende Vorrichtungen sind bekannte Linsen. Diese können die Strahlen aufweiten und fokussieren, so dass eine große Fläche mit Strahlen beaufschlagt werden kann. Eine Strahlen beeinflussende Vorrichtung ist vorzugsweise ein Scannerspiegel. Dieser ist dazu vorteilhafterweise so angeordnet und gesteuert, dass die elektromagnetischen Strahlen der Quelle als Spur auf die Messzelle und daraus folgernd auf das Bildaufnahmesystem oder den Fotodetektor gelangen. Für mehrere parallel angeordnete Spuren kann im Strahlengang vor dem Scanner ein Schwenkspiegel angeordnet sein. Zur Steuerung der Antriebe des Scanners und des eventuellen Schwenkspiegels sind diese mit dem Datenverarbeitungssystem zusammengeschaltet. Zwischen der Quelle und der strahlbeeinflussenden oder strahlformenden Vorrichtung sowie zwischen der strahlbeeinflussenden oder strahlformenden Vorrichtung und dem Fotodetektor können auch Lichtleiter angeordnet sein, so dass eine örtliche Trennung vorgenommen werden kann.

Das Bildaufnahmesystem ist eine bekannte Digitalkamera. Das abgebildete digitale Bild der Hefezellen kann über eine digitale Bildverarbeitung im Datenverarbeitungssystem bearbeitet und im Ergebnis ausgewertet werden.

Der Fotodetektor ist ein Festkörperbildsensor mit Fotowiderständen, Fotodioden oder Fototransistoren. Die daraus resultierenden Signale können direkt mittels des Datenverarbeitungssystems verarbeitet und ausgewertet werden.

### Ausführungsbeispiel 2:

Das Plasmid p426YAR071W-MFalpha1 (siehe Ausführungsbeispiel 1) zur Detektion einer Phosphorlimitierung wird direkt in einen Stamm des Paarungstyps a, z.B. den *S*. *cerevisiae-Stamm* BY4741 (*MATα; his3*Δ*1; leu2*Δ*0; met15*Δ*0; ura3*Δ*0*) transformiert.

Wird die Bildung des α-Faktors in diesem Stamm, der den entgegengesetzten Paarungstyp besitzt, induziert, wird der α-Faktor gebildet und sezerniert und die endogenen α-Faktor-Rezeptoren des Stammes aktiviert. Die a-Zellen aktivieren sich also durch den gebildeten α-Faktor selbst. Dies führt unter anderem zur beschriebenen Oberflächenpolarisierung und auch den "Shmoo"-Effekt.

In den dergestalt veränderten α-Zellen sind zudem vorteilhafterweise die authentischen chromosomalen *MFα1* und *MFα2*-Loci transkriptionsinaktiviert.

Die sensortechnische Erfassung der Zusammenlagerung der Nanopartikel, die mit gegen das Fus1p-Protein gerichtetem Antikörper funktionalisiert sind, erfolgt wie unter Ausführungsbeispiel 1 beschrieben.

## Patentansprüche

1. Einrichtung zum Nachweis einer Substanz mittels Partikel-Plasmonen-Resonanz (PPR) oder Partikel-vermittelter Fluoreszenz durch Zelloberflächenpolarisierung mit
a. Zellen, bei denen ein Gen, dessen Expression zu der polarisierten Präsentation eines Proteins auf der Oberfläche von Zellen führt, unter die Kontrolle eines durch die nachzuweisende Substanz regulierbaren Promotors gestellt ist,
b. Nanopartikeln, die mit einem Molekül funktionalisiert sind, das spezifisch an das Oberflächen-exponierte Protein binden kann,
c. wenigstens einer optischen Messeinrichtung
so dass durch Partikel-Plasmonen-Resonanz oder Partikel-vermittelte Fluoreszenz eine messbare Zusammenlagerung der Nanopartikel auf der Oberfläche der Zellen nachgewiesen werden kann.

2. Einrichtung gemäß Anspruch 1, **dadurch gekennzeichnet, dass** die Zellen Zellen eines ersten Typs und Zellen eines zweiten Typs umfassen, wobei
a. bei den Zellen des ersten Typs ein für ein Pheromon kodierendes Gen unter die Kontrolle eines durch die nachzuweisende Substanz regulierbaren Promotors gestellt ist, so dass durch das Auftreten der nachzuweisenden Substanz die Zellen des ersten Typs das Pheromon sezernieren, und
b. die Oberfläche der Zellen des zweiten Typs, die für das Pheromon responsiv sind, durch das Auftreten des Pheromons polarisiert wird.

3. Einrichtung nach Anspruch 1 oder 2, **dadurch gekennzeichnet, dass** die Zellen Hefezellen sind.

4. Einrichtung nach Anspruch 2 oder 3, **dadurch gekennzeichnet, dass** das Gen, das für ein Pheromon kodiert, das *MFα1*-Gen, das *MFα2*-Gen, das *MFA1*-Gen oder das *MFA2-Gen* ist.

5. Einrichtung nach einem der Ansprüche 2 bis 4, **dadurch gekennzeichnet, dass** die Zelle eine haploide Hefezelle ist, in der das Gen für ein Pheromon des entgegengesetzten Paarungstyps unter die Kontrolle eines Promotors gestellt ist, der durch die nachzuweisende Substanz reguliert wird.

6. Einrichtung nach einem der Ansprüche 1 bis 5, **dadurch gekennzeichnet, dass** die Nanopartikel aus Gold, Silber oder einer Legierung dieser Metalle bestehen.

7. Einrichtung nach einem der Ansprüche 1 bis 6, **dadurch gekennzeichnet, dass** das Protein, an das das spezifisch bindende Molekül bindet, Fus1p ist.

8. Einrichtung nach einem der Ansprüche 2 bis 7, **dadurch gekennzeichnet, dass** sich durch eine geeignete Wahl des Verhältnisses von Zellen des ersten Typs zu Zellen des zweiten Typs eine Signalverstärkung ergibt.

9. Einrichtung nach einem der Ansprüche 1 bis 8, **dadurch gekennzeichnet, dass** sich die Zellen in einem porösen organischen oder anorganischen Gel befinden.

10. Einrichtung nach einem der Ansprüche 1 bis 9, **dadurch gekennzeichnet, dass** die Zellen in ein Gefüge mit einer hierarchischen Porenstruktur eingebettet sind, so dass neben der für anorganische Gele typischen Nanoporosität das Gefüge zusätzlich von miteinander verbundenen Mesoporen durchzogen wird, deren Durchmesser typischerweise zwischen 10 bis 100 µm variiert und die einen Stoffaustausch zwischen der Umgebung und den eingebetteten Zellen sowie deren Reaktionsprodukten wie den Enzymen ermöglichen.

11. Einrichtung nach einem der Ansprüche 1 bis 10, **dadurch gekennzeichnet, dass** sich die Zellen wenigstens auf einer Oberfläche in einer transparenten Messzelle befinden, dass die Messzelle Einrichtungen zum Zuführen und Abführen von Medien und/oder Lösungen besitzt und dass die Messzelle mit einer Heizeinrichtung gekoppelt ist.

12. Einrichtung nach einem der Ansprüche 1 bis 11, **dadurch gekennzeichnet, dass** ein Bildaufnahmesystem als optische Messeinrichtung als eine Zellen abbildende Optik so angeordnet ist, dass eine durch die Zusammenlagerung der Nanopartikel hervorgerufene Farbänderung von Zellen als Abbildungssignale quantitativ oder qualitativ bestimmbar ist.

13. Einrichtung nach einem der Ansprüche 1 bis 12, **dadurch gekennzeichnet, dass** eine Quelle für elektromagnetische Strahlen, Zellen und mindestens eine Fotodetektor als optische Messeinrichtung so angeordnet sind, dass elektromagnetische Strahlen der Quelle auf Zellen fallen und das dadurch hervorgerufene Fluoreszenzlicht als Abbildungssignale des Fotodetektors quantitativ oder qualitativ bestimmbar ist.

14. Verfahren zum Nachweis einer Substanz mittels Partikel-Plasmonen-Resonanz (PPR) oder Partikel-vermittelter Fluoreszenz durch Zelloberflächenpolarisierung unter Nutzung von Zellen, Nanopartikeln und wenigstens einer Messeinrichtung, mit den Verfahrensschritten:
a. die Oberfläche von Zellen, in denen ein Gen, dessen Expression zu der polarisierten Präsentation eines Proteins auf der Oberfläche von Zellen führt, unter die Kontrolle eines durch die nachzuweisende Substanz regulierbaren Promotors gestellt ist, wird durch das Auftreten der Substanz polarisiert,
b. Nanopartikel, die mit einem Molekül funktionalisiert sind, das spezifisch an das Protein binden kann, das durch das Auftreten der Substanz auf der Oberfläche der Zellen polarisiert exponiert wird, binden an das Protein und
c. durch Partikel-Plasmonen-Resonanz oder Partikel-vermittelte Fluoreszenz wird mittels wenigstens einer optischen Messeinrichtung eine messbare Zusammenlagerung der Nanopartikel auf der Oberfläche der Zellen nachgewiesen.

15. Verfahren gemäß Anspruch 14, **dadurch gekennzeichnet, dass** als Zellen Zellen eines ersten Typs und Zellen eines zweiten Typs genutzt werden, wobei
a. Zellen des ersten Typs, in denen ein für ein Pheromon kodierendes Gen unter die Kontrolle eines durch die nachzuweisende Substanz regulierbaren Promotors gestellt ist, durch das Auftreten der Substanz das Pheromon sezernieren, und
b. die Oberfläche von Zellen des zweiten Typs, die für das Pheromon responsiv sind, durch das Auftreten des Pheromons polarisiert wird.

## Claims

1. Device for detecting a substance by means of particle plasmon resonance (PPR) or particle-mediated fluorescence based on cell surface polarization comprising
a. cells with a gene, the expression of which leads to the polarized presentation of a protein on the surface of cells, is under the control of a promotor that is regulatable by the substance to be detected,
b. nanoparticles that are functionalized by a molecule that can specifically bind to the surface exposed protein,
c. at least one optical measuring device
so that a measurable aggregation of the nanoparticles on the surface of the cells can be detected by means of particle plasmon resonance (PPR) or particle-mediated fluorescence.

2. Device to claim 1 **characterized by** that the cells comprise cells of a first type and cells of a second type, wherein
a. the cells of the first type comprise a gene encoding for a pheromone that is under the control of a promotor regulatable by the substance to be detected so that the cells of the first type secrete the pheromone due to the occurrence of the substance to be detected,
b. the surface of the cells of the second type, which are responsive to the pheromone, is polarized due to the occurrence of the pheromone.

3. Device to claim 1 or 2 **characterized by** that the cells are yeast cells.

4. Device to claim 2 or 3 **characterized by** that the gene that codes for a pheromone is the *MFα1*-gene, the *MFα2*-gene, the *MFA1*-gene or the *MFA2*-gene.

5. Device to one of the claims 2 to 4 **characterized by** that the cell is a haploid yeast cell in which the gene for a pheromone of the opposite mating type is placed under the control of a promotor that is regulated by the substance to be detected.

6. Device to one of the claims 1 to 5 **characterized by** that the nanoparticles consist of gold, silver or an alloy of these metals.

7. Device to one of the claims 1 to 6 **characterized by** that the protein to that the specifically binding molecule binds is Fus1p.

8. Device to one of the claims 2 to 7 **characterized by** that a signal amplification results from a suitably chosen ratio of cells of the first type to cells of the second type.

9. Device to one of the claims 1 to 8 **characterized by** that the cells are in a porous organic or inorganic gel.

10. Device to one of the claims 1 to 9 **characterized by** that the cells are embedded in a structure with a hierarchical pore system so that, in addition to the nanoporosity typical for inorganic gels, the structure is permeated by mesopores connected to each other, the diameter of which varies between 10 to 100 µm, which enable materials to be exchanged between the environment and the embedded cells as well as the reaction products thereof such as the enzymes.

11. Device to one of the claims 1 to 10 **characterized by** that the cells are situated at least at one surface in a transparent measuring cell, the measuring cell is provided with devices for feeding and removing media and/or solutions, and the measuring cell is coupled to a heating device.

12. Device to one of the claims 1 to 11 **characterized by** that an image recording system as optical measuring device is arranged as an optical system imaging cells in such manner that a colour change of cells caused by the aggregation of the nanoparticles is quantitatively or qualitatively determinable as imaging signals.

13. Device to one of the claims 1 to 12 **characterized by** that a source of electromagnetic radiation, cells and at least one photodetector as optical measuring device are arranged such that electromagnetic rays of the source fall on cells and the fluorescence light caused thereby is quantitatively or qualitatively determinable as imaging signals of the photodetector.

14. Method for detecting a substance by means of particle plasmon resonance (PPR) or particle-mediated fluorescence based on cell surface polarizations using cells, nanoparticles and at least one measuring device, comprising the process steps:
a. the surface of cells in which a gene, the expression of which leads to the polarized presentation of a protein on the surface of cells, is under the control of a promotor, which is regulatable by the substance to be detected, is polarized due to the occurrence of the substance;
b. nanoparticles that are functionalized by a molecule that can specifically bind to the protein, that is exposed polarized due to the occurrence of the substance on the surface of the cells, bind to the protein; and
c. by means of particle plasmon resonance (PPR) or particle-mediated fluorescence, a measurable aggregation of the nanoparticles on the surface of the cells is detected by at least one optical measuring device.

15. Method to claim 14 **characterized by** that cells of a first type and cells of a second type are used as cells, wherein
a. cells of the first type, in which a gene encoding for a pheromone is under the control of a promotor regulatable by the substance to be detected, secrete the pheromone due to the occurrence of the substance; and
b. the surface of cells of the second type, which are responsive to the pheromone, is polarized due to the occurrence of the pheromone.

## Revendications

1. Dispositif de détection d'une substance au moyen de la résonance plasmonique de particules (PPR) ou de la fluorescence induite par des particules par polarisation de la surface cellulaire comprenant
a. des cellules dans lesquelles un gène dont l'expression conduit à la présentation polarisée d'une protéine à la surface de cellules est placé sous le contrôle d'un promoteur pouvant être régulé par la substance à détecter,
b. des nanoparticules qui sont fonctionnalisées avec une molécule qui peut se lier spécifiquement à la protéine exposée en surface,
c. au moins un dispositif de mesure optique,
de façon que la résonance plasmonique de particules ou la fluorescence induite par des particules permette de mettre en évidence une accumulation mesurable des nanoparticules à la surface des cellules.

2. Dispositif selon la revendication 1, **caractérisé en ce que** les cellules comprennent des cellules d'un premier type et des cellules d'un second type, sachant que
a. dans les cellules du premier type, un gène codant une phéromone est placé sous le contrôle d'un promoteur pouvant être régulé par la substance à détecter, de sorte que quand la substance à détecter apparaît, les cellules du premier type sécrètent la phéromone, et
b. la surface des cellules du second type, qui sont sensibles à la phéromone, est polarisée par l'apparition de la phéromone.

3. Dispositif selon la revendication 1 ou 2, **caractérisé en ce que** les cellules sont des cellules de levure.

4. Dispositif selon la revendication 2 ou 3, **caractérisé en ce que** le gène qui code une phéromone est le gène *MFα1,* le gène *MFα2,* le gène *MFA1* ou le gène *MFA2.*

5. Dispositif selon une des revendications 2 à 4, **caractérisé en ce que** la cellule est une cellule de levure haploïde, dans laquelle le gène pour une phéromone du type sexuel opposé est placé sous le contrôle d'un promoteur qui est régulé par la substance à détecter.

6. Dispositif selon une des revendications 1 à 5, **caractérisé en ce que** les nanoparticules sont composées d'or, d'argent ou d'un alliage de ces métaux.

7. Dispositif selon une des revendications 1 à 6, **caractérisé en ce que** la protéine à laquelle se lie la molécule se liant spécifiquement est Fus1p.

8. Dispositif selon une des revendications 2 à 7, **caractérisé en ce qu'**un choix approprié du rapport de cellules du premier type et de cellules du second type permet d'obtenir une amplification des signaux.

9. Dispositif selon une des revendications 1 à 8, **caractérisé en ce que** les cellules se trouvent dans un gel poreux organique ou inorganique.

10. Dispositif selon une des revendications 1 à 9, **caractérisé en ce que** les cellules sont incorporées dans une structure dotée d'une structure poreuse hiérarchique, de sorte qu'en plus de la nanoporosité typique des gels inorganiques, la structure est traversée par des mésopores communiquant entre eux, dont le diamètre varie typiquement entre 10 et 100 µm et qui permettent un échange de matières entre l'environnement et les cellules incorporées ainsi que leurs produits de réaction comme les enzymes.

11. Dispositif selon une des revendications 1 à 10, **caractérisé en ce que** les cellules se trouvent au moins sur une surface dans une cellule de mesure transparente, que la cellule de mesure possède des dispositifs pour l'amenée et l'évacuation de milieux et/ou de solutions et que la cellule de mesure est couplée à un dispositif de chauffage.

12. Dispositif selon une des revendications 1 à 11, **caractérisé en ce qu'**un système de prise de vues sous la forme d'un dispositif de mesure optique est disposé en tant qu'optique d'imagerie des cellules de façon qu'un changement de couleur des cellules provoqué par l'accumulation des nanoparticules puisse être déterminé de manière quantitative ou qualitative sous la forme de signaux d'imagerie.

13. Dispositif selon une des revendications 1 à 12, **caractérisé en ce qu'**une source de rayons électromagnétiques, des cellules et au moins un photodétecteur sont disposés en tant que dispositif de mesure optique de façon que des rayons électromagnétiques de la source atteignent des cellules et que la lumière de fluorescence créée de cette manière puisse être déterminée de manière quantitative ou qualitative sous la forme de signaux d'imagerie du photodétecteur.

14. Procédé de détection d'une substance au moyen de la résonance plasmonique de particules (PPR) ou de la fluorescence induite par des particules par polarisation de la surface cellulaire en utilisant des cellules, des nanoparticules et au moins un dispositif de mesure, lequel procédé comprend les étapes suivantes :
a. la surface de cellules, dans lesquelles un gène dont l'expression conduit à la présentation polarisée d'une protéine à la surface de cellules est placé sous le contrôle d'un promoteur pouvant être régulé par la substance à détecter, est polarisée par l'apparition de la substance,
b. des nanoparticules qui sont fonctionnalisées avec une molécule qui peut se lier spécifiquement à la protéine qui est exposée en étant polarisée par l'apparition de la substance à la surface des cellules se lient à la protéine et
c. une accumulation mesurable des nanoparticules à la surface des cellules est détectée au moyen d'au moins un dispositif de mesure optique par résonance plasmonique de particules ou fluorescence induite par des particules.

15. Procédé selon la revendication 14, **caractérisé en ce que** l'on utilise comme cellules des cellules d'un premier type et des cellules d'un second type, sachant que
a. les cellules du premier type, dans lesquelles un gène codant une phéromone est placé sous le contrôle d'un promoteur pouvant être régulé par la substance à détecter sécrètent la phéromone quand la substance apparaît, et
b. la surface des cellules du second type, qui sont sensibles à la phéromone, est polarisée par l'apparition de la phéromone.
